# FASCICULE DE BREVET EUROPEEN

(11) **EP 0 430 831 B1**
(45) Date de publication et mention de la délivrance du brevet: **31.08.1994**
(21) Numéro de dépôt: 90420514.3
(22) Date de dépôt: 28.11.1990
(51) Int. Cl.: A61F 2/34, A61F 2/30

(54) **Cotyle artificiel à fixation par ciment dans la cavité cotyloidienne de l'os iliaque**
Künstliche Hüftgelenkpfanne zur Befestigung mit Zement in der Hüftgelenkpfannenhöhle
Artificial acetabular cup fastened in the cotyloid cavity with cement

(30) Priorité: 28.11.1989 FR 8916005
(43) Date de publication de la demande: 05.06.1991
(73) Titulaire: FABRIQUE D'IMPLANTS ET D'INSTRUMENTS CHIRURGICAUX SOCIETE A RESPONSABILITE LIMITEE, F-43240 Saint Just Malmont (FR); Winckler, Guy, F-67200 Strasbourg (FR)
(72) Inventeur: Kemps, Jean François, F-67000 Strasbourg (FR); Hencky, Pierre, 67115 Plobsheim (FR)
(74) Mandataire: Dupuis, François

(56) Documents cités:
- EP-A- 0 153 523
- EP-A- 0 235 606
- FR-A- 2 617 040
- GB-A- 2 080 118
- US-A- 4 566 138

## Description

Le principal problème rencontré avec l'implantation des implants cotyloïdiens à l'aide de ciment, réside dans la qualité de la fixation dans le temps. En effet, des études cliniques ont démontré que près de la moitié des implants cotyloïdiens ainsi implantés présentaient des signes radiologiques de descellement au bout de 10 ans. Cela est dû essentiellement à une mauvaise répartition du ciment lors de l'impaction. Notamment, l'appui de l'implant cotyloïdien implanté provoque des fluages du ciment déposé dans la cavité cotyloïdienne. La faible épaisseur de ciment à certains endroits amène dans le temps, sous l'effet des pressions exercées et des mouvements du bassin, des craquelures amorçant le descellement.

Pour tenter de remédier à ces inconvénients, on a proposé d'équiper les implants d'une structure ajourée. Cet état de la technique ressort de l'enseignement des brevets EP- A-235606 et EP-A-153523. Dans le brevet EP-A-235606, la structure ajourée prend appui dans le fond de la cavité cotyloïdienne, tandis que le ciment est situé seulement entre certaines parties de lastructure et le fond de la cavité. Dans ces conditions, la structure n'est pas totalement noyée dans l'épaisseur du ciment et ne participe pa à améliorer l'ancrage. Les mêmes inconvenients se retrouvent pour le brevet EP-A-153523, ou de plus, la structure ne présente qu'un nombre réduit d'ouvertures au travers desquelles le ciment est susceptible de passer.

L'invention s'est fixée pour but de remédier à ces inconvénients, de manière simple, sûre, efficace et rationnelle.

Le problème que se propose de résoudre l'invention est d'assurer une solidarisation sûre et durable de ce type de cotyle dans la cavité cotyloïdienne de l'os iliaque, en particulier améliorer la tenue du ciment et la bonne répartition des contraintes.

Ce problème est résolu en ce que la face extérieure de l'implant cotyloïdien présente des protubérances pour l'appui d'une structure ajourée épousant le profil externe de ladite face. Selon l'invention la structure, du type grillage en fils d'acier entrelacés notamment, est fixée sur l'implant au moyen d'organes présentant des têtes élargies de hauteur, pour s'appuyer dans le fond de la cavité cotyloïdienne, de manière à ménager, en combinaison avec la hauteur des protubérances, un espace pour noyer ladite structure dans l'épaisseur du ciment.

Avantageusement, le problème posé est résolu en ce que les protubérances sont formées directement sur la face extérieure du cotyle et sont du type bossages de hauteur déterminée, convenablement répartis sur la périphérie.

Dans une autre forme de réalisation, les protubérances sont rapportées sur la face extérieure du cotyle et sont formées par des pions étagés dont La partie inférieure est fixée dans des orifices du cotyle, tandis que la partie supérieure ou tête déborde d'une hauteur de ladite face pour l'appui de la structure.

Le problème posé de ménager dans le fond de la cavité cotyloïdienne un espace apte à recevoir la structure ajourée pour la noyer dans l'épaisseur du ciment est résolu en ce que les moyens de fixation traversent les ouvertures de la structure pour coopérer avec des orifices formés dans les protubérances, lesdits moyens présentant des têtes élargies dont la hauteur est déterminée pour s'appuyer dans le fond de la cavité cotyloïdienne.

Ce même problème est également résolu en ce que les moyens de fixation traversent les ouvertures de la structure pour coopérer avec des orifices formés dans les pions étagés, lesdits moyens présentant des têtes élargies de hauteur déterminée pour s'appuyer dans le fond de la cavité cotyloïdienne.

Avantageusement, la structure ajourée est en métal bio-compatible.

Suivant une autre caractéristique, quelle que soit la forme de réalisation du cotyle pour le montage de la structure ajourée dans les conditions indiquées, ledit cotyle présente une portée diamétrale orientée vers l'élément fémoral de la prothèse en étant assemblée par frettage à une collerette en métal biocompatible présentant un épaulement interne de retenue périphérique de la structure ajourée.

Ces caractéristiques et d'autres encore ressortiront de la description qui suit.

Pour fixer l'objet de l'invention sans toutefois le limiter dans les dessins annexés :

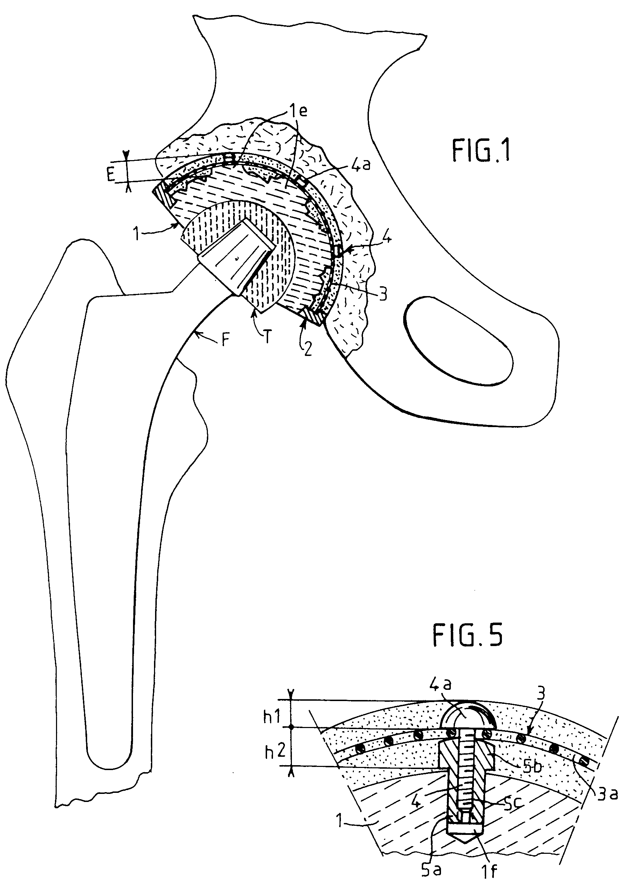

- la figure 1 est une vue d'ensemble et en coupe partielle d'une prothèse de hanche implantée avec un cotyle artificiel selon une première forme de réalisation,
- la figure 2 est une vue en coupe du cotyle selon la figure 1, équipé de la structure ajourée,
- la figure 3 est une vue extérieure du cotyle seul,
- la figure 4 est une vue en plan du cotyle seul,
- la figure 5 est une vue partielle en coupe illustrant une variante de réalisation de la fixation et de l'espacement de la structure.

Afin de rendre plus concret l'objet de l'invention, on le décrit maintenant d'une manière non limitative en se référant aux exemples de réalisation des figures des dessins.

Le cotyle (1) réalisé en polyéthylène haute densité ou autre matériau bio-compatible, a une forme générale hémisphérique avec une cavité (1a) de forme correspondante pour coopérer avec la tête (T) de l'élément fémoral (F). Une portée diamétrale (1b) est réalisée à la base pour recevoir par frettage ou autrement une collerette en métal biocompatible (2) pour l'appui contre l'os iliaque. Au-dessus de ladite portée, des rainures (1c) perpendiculaires à l'axe général permettent une meilleure interpénétration avec le ciment d'ancrage dans la cavité cotyloïdienne.

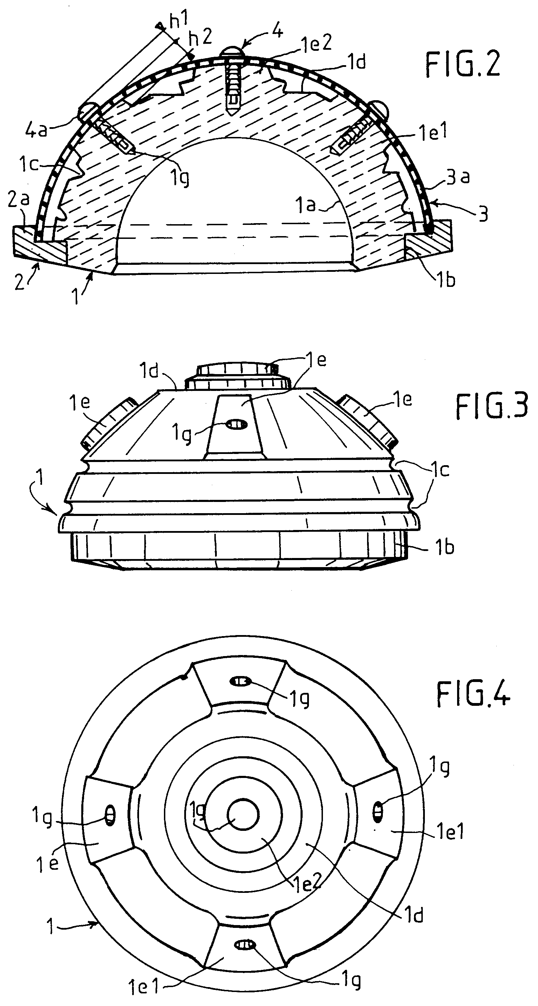

Dans la forme de réalisation illustrée, le cotyle présente une structure ajourée (3) fixée avec espacement sur la face extérieure (1d) dudit cotyle en regard de la cavité cotyloïdienne (figures 2, 3 et 4). Pour cela, le cotyle présente une pluralité de protubérances ou de bossages (1e) sur lesquels est appliquée la structure. A titre indicatif, il est prévu quatre bossages (1e1) régulièrement répartis à 90 degrés entre la dernière rainure (1c) et le sommet aplati (1d) du cotyle et un cinquième bossage (1e2) formé au centre dudit sommet.

Selon une autre forme de réalisation illustrée à la figure 5, l'espacement de la structure par rapport à la face extérieure du cotyle est obtenu par des protubérances du type pions étagés (5). Le corps (5a) des pions est fixé par tous moyens dans des orifices (1f) du cotyle, tandis que la partie supérieure ou tête (5b) desdits pions déborde de la face extérieure pour l'appui de la structure.

Suivant une autre caractéristique, la structure ajourée (3) doit être fixée par rapport au cotyle de manière à ménager dans le fond de la cavité cotyloïdienne un espace (E) déterminé pour noyer ladite structure dans l'épaisseur du ciment.

A cet effet et d'une manière avantageuse, il est prévu des moyens du type vis, rivets ou comme illustré des chevilles d'ancrage (4) traversant les ouvertures (3a) de la structure pour coopérer soit avec des orifices (1g) formés sur les bossages (1e) soit avec des orifices (5c) réalisés dans les pions étagés (5).

La tête élargie (4a) des chevilles a de préférence une surface en calotte sphérique pour s'appuyer dans la cavité cotyloïdienne. La hauteur (h1) de la tête (4a) correspond sensiblement à la hauteur (h2) des bossages (1e) ou des têtes (5b) des pions (5) de façon à ce que la structure ajourée (3) soit sensiblement au milieu de l'espace (E) délimité par la cavité cotyloïdienne et la face extérieure du cotyle entre les bossages (1e) ou les têtes (5b).

Avantageusement, la structure ajourée (3) est en métal et est obtenue à partir de fils d'acier entrelacés pour former un grillage à mailles larges ou bien en métal. Les chevilles d'ancrage passent aisément entre les mailles de la structure.

Comme on le voit bien aux figures, la structure ajourée ainsi fixée sur le cotyle avec un espacement sensiblement égal entre elle et le cotyle d'une part et entre elle et la cavité cotyloïdienne d'autre part, constitue une armature de renforcement pour le ciment augmentant de manière considérable sa résistance à la rupture par répartition homogène des contraintes. De plus, les têtes des chevilles d'ancrage constituent un auto-centrage du cotyle dans la cavité cotyloïdienne et la collerette métallique évite le contact direct polyéthylène-os. A noter encore que la structure métallique ajourée fait office de radiateur pour dissiper la chaleur dégagée par la réaction exothermique du ciment lors de sa polymérisation et d'éviter ainsi la formation de liserés à l'interface ciment-os

## Revendications

1. Implant cotyloïdien à fixation par ciment dans la cavité cotyloïdienne de l'os iliaque, la face extérieure dudit implant présentant des protubérances pour l'appui d'une structure ajourée (3) épousant le profil externe de ladite face, caractérisé en ce que la structure (3), de grillage en fils d'acier entrelacés, est fixée sur l'implant au moyen d'organes (4) présentant des têtes élargies de hauteur (h1), pour s'appuyer dans le fond de la cavité cotyloïdienne, de manière à ménager, en combinaison avec la hauteur des protubérances, un espace pour noyer ladite structure (3) dans l'épaisseur du ciment.

2. Implant selon la revendication 1, caractérisé en ce que les protubérances sont formées directement sur la face extérieure de l'implant et sont du type bossages (1e) de hauteur (h2) convenablement répartis sur la périphérie.

3. Implant selon la revendication 1, caractérisé en ce que les protubérances sont rapportées sur la face extérieure de l'implant et sont formées par des pions étagés (5) dont la partie inférieure (5a) est fixée dans des orifices (1f) de l'implant , tandis que la partie supérieure ou tête (5b) déborde d'une hauteur (h2) de ladite face pour l'appui de la structure.

4. Implant selon la revendication 1, caractérisé en ce que les moyens de fixation (4) traversent les ouvertures de la structure (3) pour coopérer avec des orifices (1g) formés dans les protubérances (1e).

5. Implant selon la revendication 1, caractérisé en ce que les moyens de fixation (4) traversent les ouvertures de la structure (3) pour coopérer avec des orifices (5c) formés dans les pions étagés (5).

6. Implant selon la revendication 1, caractérisé en ce que la structure ajourée (3) est en métal bio-compatible.

7. Implant selon la revendication 1, réalisé en matériau biocompatible du type polyéthylène haute densité, caractérisé en ce qu'une portée dimatérale (1b) orientée vers l'élément fémoral de la prothèse est assemblée par frettage à une collerette (2) en métal bio-compatible présentant un épaulement interne (2a) de retenue périphérique de la structure ajourée (3).

## Claims

1. Acetabular implant fixed with cement in the acetabulum of the iliac bone, the exterior face of said implant bearing protuberances to support an open-work structure (3) hugging the external contours of said surface, characterised in that the structure (3), a mesh of interlaced steel wires, is fixed to the implant by means of instruments (4) with enlarged heads, of height (h1), to support itself in the bottom of the acetabulum, so as to make, in combination with the height of the protuberances, a space to embed said structure (3) in the thickness of the cement.

2. Implant as claimed in claim 1, characterised in that the protuberances are formed directly on the exterior surface of the implant and are in the form of bosses (1e), of height (h2), suitably spaced on the periphery.

3. Implant as claimed in claim 1, characterised in that the protuberances are added to the exterior surface of the implant and are formed by stepped studs (5), whose lower part (5a) is fixed in the orifices (1f) of the implant, while the upper part, or head, (5b) extends above said face by a height (h2) to support the structure.

4. Implant as claimed in claim 1, characterised in that the means of fixation (4) pass through the openings in the structure (3) to engage with the orifices (1g) formed in the protuberances (1e).

5. Implant as claimed in claim 1, characterised in that the means of fixation (4) pass through the openings in the structure (3) to engage with the orifices (5c) formed in the stepped studs (5).

6. Implant as claimed in claim 1, characterised in that the open-work structure (3) is in bio-compatible metal.

7. Implant as claimed in claim 1, made from a bio-compatible material of the type high density polyethylene, characterised in that a diametrical support (1b), orientated towards the femoral element of the prosthesis, is assembled by thermofitting a bio-compatible metal collar (2) with an internal shoulder (2a) for the peripheral retention of the open-work structure (3).

## Patentansprüche

1. Gelenkpfannenimplantat zur Zementbefestigung im Azetabulum des Os ilii, wobei die Außenfläche des Implantats Vorsprünge für den Andruck einer durchbrochenen Struktur (3) aufweist, die sich an das Außenprofil der besagten Fläche anpaßt, dadurch gekennzeichnet, daß die Struktur (3), die aus einem Gitter aus ineinander verschlungenen Stahldrähten besteht, mit Vorrichtungen (4) auf dem Implantat befestigt wird, welche erweiterte Kopfteile mit der Höhe (h1) besitzen, die sich am Boden des Azetabulums abstützen, so daß in Verbindung mit der Höhe der Vorsprünge ein Raum entsteht, um die besagte Struktur (3) im Zement zu vergießen.

2. Implantat nach Anspruch 1, dadurch gekennzeichnet, daß die Vorsprünge höckerartig (1e) in der Höhe (h2) direkt aus der Außenfläche des Implantats hervorgehen und zweckmäßig an der Peripherie verteilt sind.

3. Implantat nach Anspruch 1, dadurch gekennzeichnet, daß die Vorsprünge an der Außenseite des Implantats angesetzt sind und aus abgestuften Metallstücken (5) bestehen, deren unterer Teil (5a) in Öffnungen (1f) des Implantats befestigt ist, während der obere Teil bzw. Kopf (5b) um eine Höhe (h2) zur Abstützung der Struktur über die besagte Fläche hinausragt.

4. Implantat nach Anspruch 1, dadurch gekennzeichnet, daß die Befestigungsmittel (4) die Öffnungen der Struktur (3) durchqueren, um mit Mündungen (1g) in den Vorsprüngen (1e) zusammenzuwirken.

5. Implantat nach Anspruch 1, dadurch gekennzeichnet, daß die Befestigungsmittel (4) die Öffnungen der Struktur (3) durchqueren, um mit Mündungen (5c) in den abgestuften Metallstücken (5) zusammenzuwirken.

6. Implantat nach Anspruch 1, dadurch gekennzeichnet, daß die durchbrochene Struktur (3) aus biokompatiblem Metall besteht.

7. Implantat nach Anspruch 1 aus einem biokompatiblen Werkstoff des Typs hochdichtes Polyethylen, dadurch gekennzeichnet, daß eine diametrale, zum Femurteil der Prothese hinorientierte Auflagefläche (1b) auf einen Bund (2) aus biokompatiblem Metall aufgepreßt wird, der innen einen Rücksprung (2a) zum Festhalten des Randes der durchbrochenen Struktur (3) aufweist.
